# EUROPEAN PATENT APPLICATION

(11) **EP 2 165 699 A1**
(43) Date of publication of application: **24.03.2010**
(21) Application number: 08763969.6
(22) Date of filing: 30.05.2008
(51) Int. Cl.: A61K 8/81, A61K 8/33, A61K 8/37, A61Q 5/06

(54) **HAIR COSMETIC**

(30) Priority: 31.05.2007 JP 2007146186; 20.07.2007 JP 2007189700
(71) Applicant: Kao Corporation, Chuo-Ku Tokyo 103-8210 (JP)
(72) Inventor: OSHIKA, Masato, Tokyo 131-8501 (JP); KARIYA, Naohiro, Tokyo 103-8210 (JP); SATO, Nakako, Tokyo 131-8501 (JP); NAKAMURA, Atsushi, Tokyo 131-8501 (JP)
(74) Representative: HOFFMANN EITLE
(86) International application number: PCT/JP2008/001372
(87) International publication number: WO 2008/149532

(57) **Abstract**

A hair cosmetic composition containing the following components (A) and (B): (A) is/are one or more copolymers selected from the following components (A1) and (A2):
(A1) is a copolymer of (a) from 30 to 80 wt.% of a (meth)acrylamide monomer, (b) from 2 to 50 wt.% of a (meth)acrylamide monomer, (c) from 0 to 30 wt.% of a (meth)acrylate monomer or (meth)acrylamide monomer, and (d) from 0 to 40 wt.% of a (meth)acrylate monomer;
(A2) is a copolymer of (a) from 30 to 80 wt.% a (meth)acrylamide monomer, (b) from 5 to 45 wt.% of a (meth)acrylate monomer, (c) from 2 to 30 wt.% of a (meth)acrylate monomer or (meth)acrylamide monomer, and (d) from 0 to 30 wt.% of a (meth)acrylate monomer; and
(B) is at least one selected from long-chain alkyl glyceryl ethers, fatty acid monoglycerides, fatty acid diglycerides, and fatty acid triglycerides, and

wherein the composition contains a compound having two or more hydroxyl groups in the molecule of the composition, having a molecular weight of from 62 to 1000 and being in liquid form at 30°C, in an amount less than 1 wt.%.

## Description

### [Field of the Invention]

The present invention relates to a hair cosmetic composition.

### [Background of the Invention]

Aerosol hair cosmetic compositions conventionally used for hair styling usually set the hair by making use of the fixing force of a hair styling polymer. However, the hair set once in this way cannot be styled again and those who used it feel dissatisfied with a stiff or hard feel of the hair treated therewith.
Styling agents such as hair wax hold the hair by an adhesive force of oil contained therein, so that a natural finish can be imparted without setting the hair firmly, enabling re-styling of the hair. However, the adhesive force of the oil is causative of stickiness. In addition, the adhesive force is much weaker than the fixing force of a hair styling polymer. It is therefore difficult to maintain the desired hair style for long hours.

A hair cosmetic composition containing a hair styling polymer and an oily component and therefore having an adhesive force exceeding a certain level is proposed as a composition capable of giving hair a natural hold without firmly setting the hair and has excellent hair style retention (Patent Document 1). This composition is, however, accompanied with the drawbacks that re-styling performance is low owing to insufficient adhesion so that a desired hair style cannot easily be retained for long hours and the hair cannot acquire a fluffy finish even after treatment with the composition.

In addition, there is proposed an aerosol hair cosmetic composition capable of providing a fluffy finish and ability to easily re-style the hair (Patent Document 2). It has, as a stock solution thereof, a composition containing a hair styling polymer and a polyol at a certain ratio and further containing a specific plasticizer. However, due to insufficient adhesion, the above-described hair cosmetic composition also cannot retain a desired hair style for long hours. There is therefore a demand for hair cosmetic compositions or hair styling methods superior in re-styling performance.
[Patent Document 1] JP-A-1999-116443
[Patent Document 2] JP-A-2005-68134

### [Summary of the Invention]

In the present invention, there is provided a hair cosmetic composition containing the following components (A) and (B):
(A) is one or more copolymers selected from the following (A1) and (A2):
   (A1) is a copolymer of:
      (a) from 30 to 80 wt.% of a (meth)acrylamide monomer represented by the following formula (1):

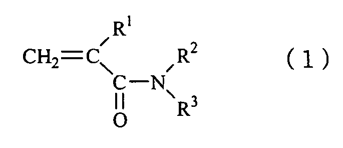

(wherein, R¹ represents a hydrogen atom or a methyl group and R² and R³ may be the same or different and each represents a hydrogen atom or an alkyl group having from 4 to 12 carbon atoms, with the proviso that R² and R³ do not represent a hydrogen atom simultaneously),
(b) from 2 to 50 wt.% of a (meth)acrylamide monomer represented by the following formula (2):

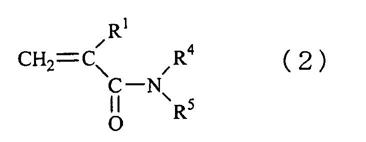

(wherein, R¹ has the same meaning as described above, R⁴ and R⁵ may be the same or different and each represents a hydrogen atom or an alkyl group having from 1 to 3 carbon atoms),
(c) from 0 to 30 wt.% of a (meth)acrylate monomer or (meth)acrylamide monomer represented by the following formula (3):

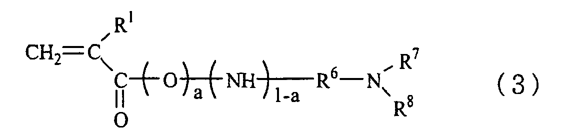

(wherein R¹ has the same meaning as described above, R⁶ represents an alkylene group having 2 or 3 carbon atoms, R⁷ and R⁸ may be the same or different and each represents a methyl or ethyl group, and a denotes a number 0 or 1), and
(d) from 0 to 40 wt.% of a (meth)acrylate monomer represented by the following formula (4):

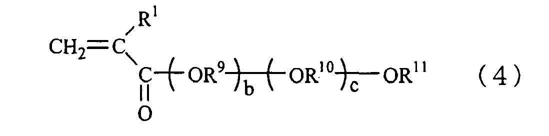

(wherein R¹ has the same meaning as described above, R⁹ and R¹⁰ may be the same or different and each represents an alkylene group having from 2 to 4 carbon atoms, R¹¹ represents a hydrogen atom, an alkyl group having from 1 to 10 carbon atoms, or a phenyl group, and b and c each denotes a number from 0 to 50, with the proviso that b and c do not stand for 0 simultaneously); and
(A2) is a copolymer of:
   (a) from 30 to 80 wt.% of a (meth)acrylamide monomer represented by the following formula (5):

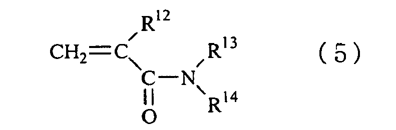

(wherein, R¹² represents a hydrogen atom or a methyl group, and R¹³ and R¹⁴ may be the same or different and each represents a hydrogen atom or an alkyl group having from 4 to 12 carbon atoms or R¹³ and R¹⁴ are coupled together to form a ring with a nitrogen atom adjacent thereto),
(b) from 5 to 45 wt.% of a (meth)acrylate monomer represented by the following formula (6):

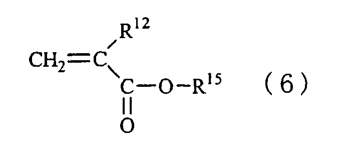

(wherein, R¹² has the same meaning as described above and R¹⁵ represents an alkyl group having from 1 to 4 carbon atoms),
(c) from 2 to 30 wt.% of a (meth)acrylate monomer or (meth)acrylamide monomer represented by the following formula (7):

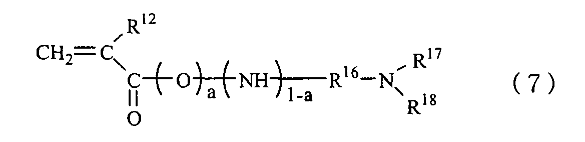

(wherein, R¹² has the same meaning as described above, R¹⁶ represents an alkylene group having 2 or 3 carbon atoms, R¹⁷ and R¹⁸ may be the same or different and each represents a methyl or ethyl group, and a denotes a number 0 or 1), and
(d) from 0 to 30 wt.% of a (meth)acrylate monomer represented by the following formula (8):

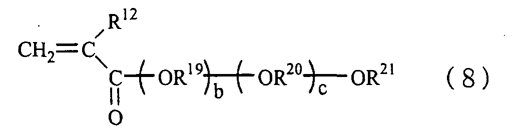

(wherein, R¹² has the same meaning as described above, R¹⁹ and R²⁰ may be the same or different and each represents an alkylene group having from 2 to 4 carbon atoms, R²¹ represents a hydrogen atom or a methyl group, and b and c each denotes a number from 0 to 50 with the proviso that b and c do not stand for 0 simultaneously); and
(B) is at least one selected from long-chain alkyl glyceryl ethers, fatty acid monoglycerides, fatty acid diglycerides, and fatty acid triglycerides.
The hair cosmetic composition contains a compound having two or more hydroxyl groups in the molecule of the composition, having a molecular weight of from 62 to 1000, and being in liquid form at 30°C, in an amount less than 1 wt.%.

In the present invention, there is also provided a hair styling method, which includes the steps of lifting up hair and spraying, to the inside thereof, an aerosol hair cosmetic composition containing (A') a hair styling polymer and (B') a plasticizer, wherein the composition has an adhesive force of 20 gf/cm² or greater when measured by the following method:

### (Measuring method)

The aerosol hair cosmetic composition is placed in a round-bottom flask. The composition is dried to constant weight at 60°C in a rotary evaporator while reducing its pressure to fall within a range of from 15 mmHg to 20 mmHg, whereby a volatile component is removed and a dry residue is obtained. A 10 wt.% solution of the dry residue in ethanol is prepared. At 25°C and 65%RH, the resulting solution is spread over a PET sheet and applied uniformly thereto by using a bar coater, followed by drying at 40°C for 60 minutes to obtain a measurement sample. After the sample is maintained at 25°C and 65%RH for 30 minutes, the adhesive force of the dry residue is measured using a tacking tester under the following conditions: 8mm diameter probe side base of being made of polypropylene, probe approaching speed of 120 mm/sec, applied pressure of 200 gf, pressure application time of 3 seconds, and pulling-up speed of the probe of 600 mm/sec.

The hair cosmetic composition of the present invention is excellent in hair styling performance and also re-styling performance and makes the hair neither sticky nor stiff. Further, the present invention makes it possible to provide hair a fluffy and light hold, retain the finished hair style for long hours, and restyle the hair even if it loses its original hair style. Moreover, it provides neither stickiness nor stiffness to the hair.

### [Detailed Description of the Invention]

The present invention relates to a hair cosmetic composition excellent in hair styling performance and also re-styling performance. The present invention also relates to a hair styling method capable of providing hair a fluffy and light hold, keeping the finished hair style for long hours, ability to re-style the hair when it loses its original hair style, and making the hair neither sticky nor stiff.

The present inventors have found that a hair cosmetic composition being excellent in hair styling performance and re-styling performance and making hair neither sticky nor stiff can be obtained by using a specific film-forming copolymer and a plasticizer component in combination. The present inventors have also found a hair styling method capable of overcoming the above-described problem, the method comprising lifting up hair and spraying, to the inside thereof, an aerosol hair cosmetic composition, the composition containing a hair styling polymer and a plasticizer and dry residue having a strong adhesive force.

Examples of the (meth)acrylamide monomer (a) represented by the formula (1) in (A1) of the copolymers as Component (A) to be used in the present invention include N-n-butyl (meth)acrylamide, N-tert-butyl (meth)acrylamide, N-octyl (meth)acrylamide, N-lauryl (meth)acrylamide, N-1-methylundecyl (meth)acrylamide, N-2-ethylhexyl (meth)acrylamide, and N-tert-octyl (meth)acrylamide. Of these, N-(branched alkyl) (meth)acrylamides such as N-tert-butyl (meth)acrylamide, N-tert-octyl (meth)acrylamide, and N-2-ethylhexyl (meth)acrylamide are more preferred.
They may be used alone or in combination in an amount of from 30 to 80 wt.%, preferably from 40 to 70 wt.% based on the total amount of the monomers.

Examples of the (meth)acrylamide monomer (b) represented by the formula (2) include (meth)acrylamide, N-methyl (meth)acrylamide, N-ethyl (meth)acrylamide, N-isopropyl (meth)acrylamide, N,N-dimethyl (meth)acrylamide, and N,N-diethyl (meth)acrylamide. Of these, N-methyl (meth)acrylamide, N-ethyl (meth)acrylamide, N,N-dimethyl (meth)acrylamide, N,N-diethyl (meth)acrylamide, and the like are more preferred.
They may be used alone or in combination in an amount of from 2 to 50 wt.%, preferably from 10 to 35 wt.% based on the total amount of the monomers.

Examples of the (meth)acrylate and (meth)acrylamide monomer (c) represented by the formula (3) include N,N-dimethylaminoethyl (meth)acrylate, N,N-diethylaminoethyl (meth)acrylate, N,N-dimethylaminopropyl (meth)acrylate, N,N-dimethylaminopropyl (meth)acrylamide, and N,N-diethylaminopropyl (meth)acrylamide.
They may be used alone or in combination in an amount of from 0 to 30 wt.%, preferably from 0 to 10 wt.%, more preferably from 0.5 to 5 wt.% based on the total amount of the monomers.

The monomer (d) represented by the formula (4) is a (meth)acrylate having a polyoxyalkylene chain. In the formula, R¹¹ represents a hydrogen atom, an alkyl group having from 1 to 10 carbon atoms, or a phenyl group, preferably a hydrogen atom or an alkyl group having from 1 to 4 carbon atoms, more preferably a methyl group. Examples of such a (meth)acrylate monomer (4) include hydroxyethyl (meth)acrylate, hydroxypropyl (meth)acrylate, polyethylene glycol mono(meth)acrylate, polypropylene glycol mono(meth)acrylate, methoxy polyethylene glycol mono(meth)acrylate, methoxy polypropylene glycol mono(meth)acrylate, ethoxy polyethylene glycol mono(meth)acrylate, butoxy polyethylene glycol mono(meth)acrylate, and phenoxy polyethylene glycol mono(meth)acrylate. The polyoxyalkylene chain is a homopolymer or copolymer of a C₂₋₄ alkylene oxide. When it is the copolymer, it may be either a block copolymer or a random copolymer of ethylene oxide, propylene oxide, or the like. The degree of polymerization of the alkylene oxide can be analyzed by gas chromatography and it is preferably from 1 to 50 on average.
They may be used alone or in combination in an amount of from 0 to 40 wt.%, preferably from 5 to 30 wt.%, more preferably from 10 to 25 wt.% based on the total amount of the monomers.

Preferred examples of the copolymer as (A1) include N-tert-butyl (meth)acrylamide/N,N-dimethyl (meth)acrylamide/N,N-dimethylaminopropyl (meth)acrylamide/methoxyl polyethylene glycol (meth)acrylate, N-tert-butyl (meth)acrylamide/N,N-dimethyl (meth)acrylamide/N,N-dimethylaminoethyl (meth)acrylate/methoxyl polyethylene glycol (meth)acrylate, N-tert-butyl (meth)acrylamide/N-methyl (meth)acrylamide/N,N-dimethylaminoethyl (meth)acrylate/methoxyl polyethylene glycol (meth)acrylate, N-tert-butyl (meth)acrylamide/N-methyl (meth)acrylamide/N,N-dimethylaminopropyl (meth)acrylamide/methoxyl polyethylene glycol (meth)acrylate, N-tert-butyl (meth)acrylamide/N,N-dimethyl (meth)acrylamide/N,N-dimethylaminopropyl (meth)acrylamide, N-tert-octyl (meth)acrylamide/N,N-diethyl (meth)acrylamide/N,N-dimethylaminopropyl (meth)acrylamide/methoxyl polyethylene glycol (meth)acrylate, N-tert-octyl (meth)acrylamide/N,N-dimethyl (meth)acrylamide/N,N-dimethylaminopropyl (meth)acrylamide/methoxyl polyethylene glycol (meth)acrylate, N-tert-butyl (meth)acrylamide/N,N-dimethyl (meth)acrylamide/N,N-dimethylaminopropyl (meth)acrylamide/2-hydroxyethyl (meth)acrylate, and N-tert-butyl (meth)acrylamide/N,N-diethyl (meth)acrylamide/N,N-dimethylaminopropyl (meth)acrylamide/methoxyl polyethylene glycol (meth)acrylate. Of these, N-tert-butyl (meth)acrylamide/N,N-dimethyl (meth)acrylamide/N,N-dimethylaminopropyl (meth)acrylamide/methoxyl polyethylene glycol (meth)acrylate is more preferred.

Examples of the (meth)acrylamide monomer (a) represented by the formula (5) in (A2) include (meth)acrylamide, N-n-butyl (meth)acrylamide, N-tert-butyl (meth)acrylamide, N-octyl (meth)acrylamide, N-lauryl (meth)acrylamide, and (meth)acryloylmorpholine. Of these, N-butyl (meth)acrylamide, N-octyl(meth)acrylamide, and N-lauryl (meth)acrylamide are more preferred.
They may be used alone or in combination in an amount of from 30 to 80 wt.%, preferably from 40 to 70 wt.% based on the total amount of the monomers.

Examples of the (meth)acrylate monomer (b)represented by the formula (6) include methyl (meth)acrylate, ethyl (meth)acrylate, propyl (meth)acrylate, and butyl (meth)acrylate.
They may be used alone or in combination in an amount of from 5 to 45 wt.%, preferably from 10 to 40 wt.% based on the total amount of the monomers.

Examples of the (meth)acrylate and (meth)acrylamide monomer (c) represented by the formula (7) include N,N-dimethylaminoethyl (meth)acrylate, N,N-diethylaminoethyl (meth)acrylate, N,N-dimethylaminopropyl (meth)acrylate, N,N-dimethylaminopropyl (meth)acrylamide, and N,N-diethylaminopropyl (meth)acrylamide.
They may be used alone or in combination in an amount of from 2 to 30 wt.%, preferably from 5 to 20 wt.% based on the total amount of the monomers.

Examples of the (meth)acrylate monomer (d) represented by the formula (8) include hydroxyethyl (meth)acrylate, hydroxypropyl (meth)acrylate, polyethylene glycol mono(meth)acrylate, polypropylene glycol mono(meth)acrylate, methoxyl polyethylene glycol mono(meth)acrylate, and methoxyl polypropylene glycol mono(meth)acrylate. The polyoxyalkylene chain is a homopolymer or copolymer of a C₂₋₄ alkylene oxide. When it is the copolymer, it may be either a block copolymer or a random copolymer of ethylene oxide, propylene oxide, or the like. The degree of polymerization of the alkylene oxide can be analyzed by gas chromatography and it is preferably from 1 to 50 on average.
They may be used alone or in combination in an amount of from 0 to 30 wt.%, preferably from 5 to 15 wt.% based on the total amount of the monomers.

Preferred examples of the copolymer (A2) include N-tert-butyl (meth)acrylamide/N,N-dimethylaminopropyl (meth)acrylamide/methoxyl polyethylene glycol (meth)acrylate/ethyl (meth)acrylate, N-tert-butyl (meth)acrylamide/N,N-dimethylaminoethyl (meth)acrylate/methoxyl polyethylene glycol (meth)acrylate/ethyl (meth)acrylate, N-tert-butyl (meth)acrylamide/N,N-dimethylaminoethyl (meth)acrylate/methoxyl polyethylene glycol (meth)acrylate/methyl (meth)acrylate, N-tert-butyl (meth)acrylamide/N,N-diethylaminoethyl (meth)acrylate/methoxyl polyethylene glycol (meth)acrylate/ethyl (meth)acrylate, N-tert-butyl (meth)acrylamide/N,N-dimethylaminoethyl (meth)acrylate/2-hydroxyethyl (meth)acrylate/ethyl (meth)acrylate, N-tert-octyl (meth)acrylamide/N,N-dimethylaminoethyl (meth)acrylate/methoxyl polyethylene glycol (meth)acrylate/n-butyl (meth)acrylate, N-tert-octyl (meth)acrylamide/N,N-diethylaminoethyl (meth)acrylate/methoxyl polyethylene glycol (meth)acrylate/n-butyl (meth)acrylate, and N-tert-butyl (meth)acrylamide/N,N-dimethylaminopropyl (meth)acrylamide/ethyl (meth)acrylate. Of these, N-tert-butyl (meth)acrylamide/N,N-dimethylaminopropyl (meth)acrylamide/methoxyl polyethylene glycol (meth)acrylate/ethyl (meth)acrylate is more preferred.

The copolymers (A1) and (A2) can each be prepared in accordance with the process as described in, for example, JP-A-1996-291206 or JP-A-1990-180911 by using the above-described monomers in combination.

The weight-average molecular weight (as measured by gel filtration chromatography (polyethylene glycol standards)) of the resulting copolymers can be controlled to from 1,000 to 1,000,000, depending on selected polymerization conditions. In the present invention, the copolymers have preferably a weight average molecular weight of from 10,000 to 500,000, more preferably from 20,000 to 200,000.

The thus-obtained copolymers can be used after neutralization of their tertiary amino group with an inorganic acid or an organic acid in order to provide water solubility to them. In this case, it is preferred to neutralize 50% or more of all of their tertiary amino groups.

Examples of the inorganic acid include hydrochloric acid, sulfuric acid, and phosphoric acid, while those of the organic acid include acetic acid, glycolic acid, dimethyl glycolic acid, lactic acid, dimethylolpropionic acid, tartaric acid, citric acid, maleic acid, and malic acid.

The amino group in the copolymer may be quaternized with a proper quaternizing agent. In this case, it is preferred to quaternize 50% or more of all the tertiary amino groups.

Examples of such a quaternizing agent include dialkyl sulfates such as dimethyl sulfate and diethyl sulfate, and alkyl halides or aralkyl halides such as methyl chloride, propyl bromide, and benzyl chloride.

Such a quaternized copolymer is also available by quaternizing the monomer (3) or (7) with the quaternizing agent, followed by copolymerization.

As Component (A), one or more copolymers selected from (A1) and (A2) can be used. It is preferably contained in an amount of from 0.3 to 10 wt.%, more preferably from 0.5 to 5 wt.%, even more preferably from 0.7 to 3.5 wt.% based on the total amount of the hair cosmetic composition (the total amount including a propellant when the composition contains the propellant, hereinafter the same) from the standpoint of achieving high styling power and re-styling performance.

Component (B) is selected from long-chain alkyl glyceryl ethers, fatty acid monoglycerides, fatty acid diglycerides, and fatty acid triglycerides.

The long-chain alkyl group of the long-chain alkyl glyceryl ethers may be either a linear or branched one, but a branched one is more preferred. The number of carbon atoms of the long-chain alkyl group is preferably from 4 to 22, more preferably from 8 to 18. Specific examples include 2-ethylhexyl group, octyl group, decyl group, isodecyl group, lauryl group, cetyl group, stearyl group, isostearyl group, and behenyl group, with isodecyl group, lauryl group, cetyl group, and isostearyl group being preferred.

As the fatty acid of the fatty acid monoglycerides, fatty acid diglycerides, and fatty acid triglycerides, those having from 4 to 22 carbon atoms are preferred, with those having from 8 to 18 carbon atoms being more preferred. Specific examples include caproic acid, caprylic acid, capric acid, lauric acid, palmitic acid, palmitoyl acid, stearic acid, isostearic acid, linoleic acid, oleic acid, and behenic acid.
Monoglycerides, diglycerides or triglycerides of these fatty acids are preferred. Specific examples include oleic acid monoglyceride, caprylic acid monoglyceride, capric acid monoglyceride, oleic acid diglyceride, caprylic acid diglyceride, capric acid diglyceride, oleic acid triglyceride, caprylic acid triglyceride, and capric acid triglyceride.

At least one of them may be used as Component (B). Component (B) is contained in an amount of preferably from 0.5 to 15 wt.%, more preferably from 0.8 to 8 wt.%, even more preferably from 1 to 5 wt.% based on the total amount of the hair cosmetic composition from the standpoint of plasticizing Component (A) to induce high adhesive force.

When two or more compounds are used in combination as Component (B), they contain preferably at least one or both of the fatty acid monoglyceride and alkyl glyceryl ether, more preferably both of them. In this case, the sum of the fatty acid monoglyceride and the alkyl glyceryl ether is preferably 45 wt.% or greater, more preferably 55 wt.% or greater, even more preferably 65 wt.% or greater in the total amount of Component (B).

A weight ratio of Component (A) to Component (B), that is, a (A)/(B) weight ratio is adjusted to fall within a range of preferably from 65/35 to 35/65, more preferably from 55/45 to 40/60 from the standpoint of achieving high hair styling performance and re-styling performance.

The hair cosmetic composition of the present invention further contains ethanol and/or water. The content of them is preferably from 4 to 98.4 wt.%, more preferably from 30 to 98 wt.%, even more preferably from 50 to 95 wt.% in the whole composition from the standpoint of improving the solubility of the copolymer or plasticizer in the hair cosmetic composition or improving the spray condition.

The hair cosmetic composition of the present invention contains a compound having, in the molecule thereof, two or more hydroxyl groups, having a molecular weight of from 62 to 1000, preferably from 62 to 2000, more preferably from 62 to 3000, most preferably 62 or greater, and being in liquid form at 30°C, in an amount less than 1 wt.%, preferably less than 0.5 wt.%, more preferably zero.
Examples of such a compound include propylene glycol (molecular weight: 76.1), 1,3-butylene glycol (molecular weight: 90.1), glycerin (molecular weight: 92.1), isopentylene glycol (molecular weight: 104.1), hexylene glycol (molecular weight: 118.2), dipropylene glycol (molecular weight: 134.2), polypropylene glycol (degree of polymerization: 9, molecular weight: 540), and polyethylene glycol 600. The term "liquid form" as used herein means that the compound has a viscosity, as measured using a Brookfield rotary viscometer (Rotor No. 2, rotation at 12 rpm for 60 seconds at 30°C) not greater than 1000 mPa·s.

The hair cosmetic composition of the present invention may contain various components used for conventional hair cosmetic compositions such as cationic surfactants, anionic surfactants, amphoteric surfactants, pH regulators, vitamin preparations, proteins, amino acids, crude drugs, antiseptics, ultraviolet absorbers, antioxidants, and colorants, depending on the intended use.
The hair cosmetic composition of the present invention can be used for an aerosol spray, pump spray, aerosol foam, pump foam, hair wax, setting lotion, and the like.

The hair cosmetic composition of the present invention can also be provided as an aerosol hair cosmetic composition by adding thereto (C) a propellant.
A weight ratio of components other than Component (C) (hereinafter referred to as "stock solution") to Component (C), that is, the weight ratio of a (stock solution)/(C) is preferably from 50/50 to 30/70, more preferably from 45/55 to 35/65 from the viewpoint of facilitating workability and achieving hair styling performance and re-styling performance.

The aerosol hair cosmetic composition can be prepared by filling a pressure bottle with the stock solution and the propellant.

The stock solution has a viscosity at 30°C of preferably 15 mPa·s or less, more preferably 10 mPa·s or less in order to spray the stock solution as fine droplets. The term "viscosity" as used herein means a value measured using a Brookfield viscometer (rotor with BL adaptor, rotation at 30 rpm, for 60 seconds, and at 30°C).

Examples of the propellant include liquefied natural gas (LPG), dimethyl ether (DME), carbon dioxide gas, nitrogen gas, and mixtures thereof. Alternatives for chlorofluorocarbon such as HFC-152a may be used. With regard to the amount of the propellant, a weight ratio of the stock solution to the propellant, that is, the weight ratio of a (stock solution)/propellant, is preferably from 5/95 to 80/20, more preferably from 40/60 to 70/30 in order to achieve good spraying characteristics and good adhesion characteristics. In addition, the pressure in the pressure bottle is adjusted to preferably from 0.12 to 0.45 MPa at 25°C in order to achieve good spraying characteristics and good adhesion characteristics.

A valve to be used for the pressure bottle has preferably the stem hole diameter ϕ of from 0.33 to 0.46 mm and the bottom hole diameter of a housing ϕ from 0.33 to 0.65 mm x the side hole diameter of a housing ϕ of from 0 to 0.64 mm. In a water-containing formulation, the valve has more preferably the stem hole diameter ϕ of 0.33 to 0.42 mm and the bottom hole diameter of a housing ϕ of from 0.33 to 0.42 mm but has no side hole, while in a nonaqueous formulation, the valve has more preferably the stem hole diameter ϕ of from 0.40 to 0.46 mm and the bottom hole diameter of a housing ϕ of from 0.42 to 0.65 mm x the side hole diameter of a housing ϕ of from 0.33 to 0.46 mm.

In the aerosol hair cosmetic composition to be used in the hair styling method of the present invention, examples of the hair styling polymer as Component (A') include the alkyl acrylamide/acrylate/alkylaminoalkyl acrylamide/polyethylene glycol methacrylate copolymers as described in JP-A-1990-180911, alkyl acrylamide/alkylaminoalkyl acrylamide/polyethylene glycol methacrylate copolymers as described in JP-A-1996-291206, (methacryloyloxyethylcarboxybetaine/alkyl methacrylate) copolymers such as "Yukaformer R205" (product of Mitsubishi Chemical) and "RAM Resin" (product of Osaka Organic Chemical Industry), (acrylates/lauryl acrylate/stearyl acrylate/ethylamine oxide methacrylate) copolymers such as "Diaformer Z-712" (product of Mitsubishi Chemical), (vinylamine/vinyl alcohol) copolymers such as "Diafix C-601" (product of Mitsubishi Chemical), (alkyl acrylate/diacetonacrylamide) copolymers neutralized with aminomethyl propanol such as "Plas Cize L-9540B" (product of Goo Chemical), acrylic acid/acrylic acid amide/ethyl acrylate copolymers such as "Ultrahold 8" and "Ultrahold Strong" (each, product of BASF), alkyl acrylate/methacrylic acid/silicone copolymer such as "Luviflex Silk" (product of BASF), polyurethane such as "Luviset P.U.R." (product of BASF), polyvinylcaprolactam such as "Luviskol Plus" (product of BASF), alkyl acrylate copolymers such as "Luvimer 100P" and "Luvimer 30E" (each, product of BASF), (octyl acrylamide/hydroxypropyl acrylate/butylaminoethyl methacrylate) copolymers such as "Amphomer SH-701", "Amphomer 28-4910", "Amphomer LV-71" and "Amphomer LV-47" (each, product of National Starch & Chemicals), (alkyl acrylate/octylacrylamide) copolymers such as "Amphomer V-42" (product of National Starch & Chemicals), (vinyl acetate/crotonic acid/vinyl neodecanoate) copolymers such as "Resyn 28-2930" (product of National Starch & Chemicals), polyurethane-14·AMP-acrylates copolymers such as "DynamX" (product of National Starch & Chemicals), diethyl sulfate quaternized (vinylpyrrolidone/dimethylaminoethyl methacrylate) copolymers (Polyquaternium-11) such as "Gafquat 440" (product of ISP), methyl quaternized vinylpyrrolildone/dimethylaminopropyl methacrylamide (Polyquaternium-28) such as "Gafquat HS-100" (product of ISP), (vinyl methyl ether/ethyl maleate) copolymers such as "Gantrez ES-225" (product of ISP), (PVP/vinyl caprolactam/DMAPA acrylate) copolymers such as "Aquaflex SF-40" (product of ISP), (isobutylene/ethylmaleimide/hydroxyethylmaleimide) copolymers such as "Aquaflex FX-64" (product of ISP), (vinylpyrrolidone/dimethyamino propylmethacrylamide/methacryloylaminopropyl lauryl dimethyl ammonium chloride) copolymers (Polyquaternium-55) such as "Styleze w-20" (product of ISP), (vinylpyrrolidone/DMAPA acrylate) copolymers such as "Styleze CC-10" (product of ISP), and (vinylpyrrolidone/vinyl acetate) copolymers such as "PVP/VA735" (product of ISP) and "Luviskol VA64P (product of BASF).

Of the above-described hair styling polymers, alkyl acrylamide/acrylate/alkylaminoalkyl acrylamide/polyethylene glycol methacrylate copolymers, alkyl acrylamide/alkylaminoalkyl acrylamide/polyethylene glycol methacrylate copolymers, (methacryloyloxyethylcarboxybetaine/alkyl methacrylate) copolymers, (alkyl acrylate/diacetoneacrylamide) copolymers neutralized with aminomethylpropanol, (octyl acrylamide/hydroxypropyl acrylate/butylaminoethyl methacrylate) copolymer, acrylic acid/acrylic acid amide/ethyl alkylate copolymers, polyvinylcaprolactam, and (alkyl acrylate/octylacrylamide) copolymers are preferred, with alkyl acrylamide/acrylate/alkylaminoalkyl acrylamide/polyethylene glycol methacrylate copolymers and alkyl acrylamide/alkylaminoalkyl acrylamide/polyethylene glycol methacrylate copolymers being more preferred.

As Component (A'), two or more of these hair styling polymers can be used in combination. Its content in the stock solution of the aerosol hair cosmetic composition preferably ranges from 0.5 to 10 wt.%, more preferably from 1 to 8 wt.%, even more preferably 1.5 to 7 wt.% in order to provide hair a fluffy and light finish by spot bonding of individual hairs and maintaining the thus-achieved style.

As the plasticizer of Component (B'), solvents having, in the molecule thereof, a hydroxyl group, nonionic surfactants, ester oils, and N-hydroxyethylcarboxylic acid amide can be used.

Specific examples of the solvents having, in the molecule thereof, a hydroxyl group include monohydric alcohols such as benzyl alcohol, ethoxyethyl alcohol, and phenoxyethanol; and polyhydric alcohols such as propylene glycol, 1,3-butylene glycol, glycerin, isopentylene glycol, hexylene glycol, dipropylene glycol, polypropylene glycol (degree of polymerization: 9), and polyethylene glycol 600.

Specific examples of the nonionic surfactant include fatty acid monoglycerides such as oleic acid monoglyceride and caprylic acid monoglyceride; fatty acid diethanolamides such as palm kernel fatty acid diethanolamide; sorbitan fatty acid esters such as sorbitan monooleate and sorbitan monolaurate; polyoxyalkylene sorbitan fatty acid esters such as polyoxyethylene sorbitan trioleate, polyoxyethylene sorbitan monooleate, polyoxyethylene sorbitan monostearate, and polyoxyethylene sorbitan monopalmitate; alkyl glyceryl ethers such as isostearyl glyceryl ether and isodecyl glyceryl ether; fatty acid polyoxyethylene sorbitols such as polyoxyethylene sorbitol tetraoleate and polyoxyethylene sorbitol tetraoleate; and polyoxyalkylene alkyl ethers such as polyoxyethylene (9) tridecyl ether and polyoxyethylene (9) lauryl ether.

Examples of the ester oil include fatty acid triglycerides such as (caprylic/capric acid) triglyceride and oleic acid triglyceride; fatty acid esters of a divalent alcohol such as neopentyl glycol dipalmitate and (hydroxystearic acid/stearic acid/rosic acid)dipentaerythrityl; and fatty acid esters of a monohydric alcohol such as isopropyl myristate.
Examples of the N-hydroxyethylcarboxylic acid amide include N-acetylethanolamide.

As the plasticizer which is Component (B'), the above-described nonionic surfactants are preferred because they have high compatibility with the styling polymer which is amphipathic so that even after application to the hair, neither separation nor loss due to evaporation occurs and can therefore keep a good plasticizing effect on the hair styling polymer. Of the above-described nonionic surfactants, fatty acid monoglycerides such as oleic acid monoglyceride and caprylic acid monoglyceride; sorbitan fatty acid esters such as sorbitan monooleate and sorbitan monolaurate; polyoxyalkylene sorbitan fatty acid esters such as polyoxyethylene sorbitan trioleate, polyoxyethylene sorbitan monooleate, polyoxyethylene sorbitan monostearate, and polyoxyethylene sorbitan monopalmitate; alkyl glyceryl ethers such as isostearyl glyceryl ether and isodecyl glyceryl ether; fatty acid polyoxyethylene sorbitols such as polyoxyethylene sorbitol tetraoleate and polyoxyethylene sorbitol tetraoleate; and polyoxyalkylene alkyl ethers such as polyoxyethylene (9) tridecyl ether and polyoxyethylene (9) lauryl ether are more preferred. Of these, fatty acid monoglycerides such as oleic acid monoglyceride and caprylic acid monoglyceride and alkyl glyceryl ethers such as isostearyl glyceryl ether and isodecyl glyceryl ether are most preferred from the standpoint of keeping the adhesive force of the hair styling polymer at the highest level.

The content of the plasticizer as Component (B') in the stock solution of the aerosol hair cosmetic composition is preferably from 1 to 25 wt.%, more preferably from 2 to 10 wt.%, even more preferably from 4 to 8 wt.% in order to sufficiently plasticize the hair styling polymer and sufficiently ensuring the adhesive force generated by the plasticization.

Two or more of the above-described plasticizers may be used in combination as Component (B'). In this case, they contain preferably at least either one or both of the fatty acid monoglyceride and the alkyl glyceryl ether. When both of them are contained, the sum of the fatty acid monoglyceride and the alkyl glyceryl ether is 45 wt.% or greater, more preferably 55 wt.% or greater, even more preferably 65 wt.% or greater in the total amount of the plasticizers.

A mass ratio of Component (A') to Component (B'), that is, a weight ratio of (A')/(B') is adjusted to preferably from 0.42 to 2.3, more preferably from 0.47 to 2.1, even more preferably from 0.52 to 1.9 in order to provide an adhesion state under which a fluffy and light finish and ability to easily re-style hair can be achieved.

The stock solution of the aerosol hair cosmetic composition further contains ethanol and/or water. The content of ethanol and/or water in the stock solution is preferably from 4 to 98.4 wt.%, more preferably from 30 to 98 wt.%, even more preferably from 50 to 95 wt.% from the standpoint of improving the solubility of the hair styling polymer or plasticizer contained in the stock solution or improving the spray condition.

In the stock solution of the aerosol hair cosmetic composition similar to the above, various components to be used for conventional hair cosmetic compositions can be incorporated, depending on the intended use.

The stock solution has, similar to the above-described stock solution, a viscosity at 30°C of preferably 15 mPa·s or less, more preferably 10 mPa·s or less.

The aerosol hair cosmetic composition is prepared by filling a pressure bottle with such a stock solution and propellant.
As the propellant, those similar to the above-described ones are used preferably at the same mass ratio. As a valve to be used for the pressure bottle, that similar to the above-described one is preferred.

In the aerosol hair cosmetic composition, the adhesive force of a dry residue as measured by the below-described method is preferably 20 gf/cm² or greater, more preferably from 20 gf/cm² to 500 gf/cm², even more preferably from 40 gf/cm² to 400 gf/cm², even more preferably from 60 gf/cm² to 300 gf/cm², even more preferably from 80 gf/cm² to 200 gf/cm², from the viewpoint of a fluffy and light hold and durability of a hair style.

### (Measuring method)

The aerosol hair cosmetic composition is placed in a round-bottom flask. The composition is dried to constant weight at 60°C in a rotary evaporator while reducing pressure to fall within a range of from 15 mmHg to 20 mmHg, whereby a volatile component is removed and a dry residue is obtained. A 10 wt.% solution of the dry residue in ethanol is prepared. At 25°C and 65%RH, the resulting solution is spread over a PET sheet and applied uniformly thereto by using a bar coater, followed by drying at 40°C for 60 minutes to obtain a measurement sample. After the sample is maintained at 25°C and 65%RH for 30 minutes, the adhesive force of the dry residue is measured using a tacking tester under the following conditions: 8mm diameter probe side base of being made of polypropylene,, probe approaching speed of 120 mm/sec, applied pressure of 200 gf, pressure application time of 3 seconds, and pulling-up speed of the probe of 600 mm/sec.
In the invention, as the PET sheet, "MINERON A-PET Code 506M" manufactured by Mineron Kasei is used. As the bar coater, a bar coater No. 120 manufactured by Daiichi Rika is used and application is performed at a rate of 2 cm/sec. In the present invention, four measurement samples are prepared for one dry residue and the adhesive force is measured at five different points. This means that the adhesive force is measured at 20 points in total and an average is employed as a measurement value.

The aerosol hair cosmetic composition may be obtained by spraying the aerosol hair cosmetic composition into the round-bottom flask and collecting the thus-sprayed solution. No limitation is imposed on the spraying time or amount.
The volatile components removed by using the rotary evaporator are solvent, propellant, and the like. The dry residue contains the hair styling polymer (A'), the plasticizer (B'), an alkali, an acid, a perfume, a salt, and the like.

A 10 wt.% ethanol solution of the dry residue thus prepared is uniformly spread and applied to the sheet at a rate of 2 cm/sec by using "Bar Coater No. 120" manufactured by Daiichi Rika, followed by drying at 40°C for 60 minutes. This makes it possible to apply the solution to the sheet to provide a dry residue of about 30 mg/cm². As a tacking tester, "TAC II UC-2006" manufactured by Rhesca Corporation may be used.

The solution (stock solution) sprayed from the bottle of the aerosol hair cosmetic composition preferably forms fine liquid droplets having an average particle size (sprayed particle size; 50% value of the cumulative volume distribution), at a measurement distance (distance from an spraying port) of 20 cm, of preferably from 15 to 60 µm, more preferably from 20 to 45 µm from the standpoint of spot bonding of individual hairs to provide them a fluffy and light finish. The term "average particle size of the fine liquid droplets" as used herein means 50% of the cumulative volume distribution as measured (measurement distance: 15 cm) by spraying the solution directly to laser light at a measuring range R4 (focus distance: 200 mm, particle size measuring range from 0.5/1.8 to 350 µm) by using a laser diffraction particle size distribution analyzer "HELOS SYSTEM" manufactured by Sympatec GmbH, system-Partikel-technik.

An spraying amount of the aerosol hair cosmetic composition is preferably from 2 to 7 g, more preferably from 2.5 to 4.5 g from the standpoint of achieving a sufficient hair styling performance and preventing the hair from become sticky.
The term "spraying amount" as used herein means a decrease in mass of the whole spray after the aerosol composition is sprayed by pushing a spraying button of the spray continuously for 10 seconds.

In the present invention, hair styling is performed using such an aerosol hair cosmetic composition. The term "hair styling" as used herein means the styling of hair, more specifically, fixing the hair to a hair style with a fluffy and light hold. The term "hair setting" also has the same meaning and is usually employed.
After hair is lifted up, the aerosol hair cosmetic composition is sprayed to the inside of the lifted-up hair. The term "inside of the lifted-up hair" as used herein means a portion of the lifted-up hair which has existed on the scalp side before it is lifted up. It is preferred to lift up the hair by holding it with portions of fingers from the tip to the first joint thereof, and is also preferred to hold and lift up the hair divided into small bundles. More specifically, it is preferred to adjust the diameter of the hair bundle to be held with fingers at one time to from 5 to 20 mm in order to carry out efficient hair styling and uniformly and sufficiently spray the composition to the inside of the hair bundle.

Further, it is preferred to spray the aerosol hair cosmetic composition to dry hair, that is, hair having a water content not greater than a saturated water content of the hair in order to retain the hair style for long hours and achieve a fluffy and light finish.
It is also preferred to spray the composition to the hair at a portion of 2 cm or more, more preferably 5 cm or more distant from the scalp on the hair tip side in order to provide the hair with a fluffy and light hold.

The spraying amount is adjusted so that a dry residue of a solution to be applied to the hair becomes preferably from 3 to 20 mg/cm² and it is more preferably from 5 to 15 mg/cm², even more preferably from 7 to 12 mg/cm² because within the above range, a hair style with a non-sticky and fluffy hold can be formed readily. In addition, spraying from a position preferably 10 to 30 cm, more preferably 15 to 20 cm distant from the hair is preferred in order to form a non-sticky and fluffy hair style easily. With regard to the amount of the dry residue of the composition sprayed to hair, by using a relation, determined in advance, between an increase in the mass and an spraying amount when the composition is sprayed to a glass plate and dried at 40°C for 60 minutes, the amount of the dry residue is calculated from the spraying amount. It is to be noted that the calculation is made by assuming that all the amount of the dry residue thus sprayed is applied to the hair.

A spraying operation is preferably performed to disperse the drying residue of the application solution. When the solution is sprayed without moving the spray bottle, it is preferred to push a spray button continuously for 0.2 to 2 sec/single push, preferably for from 0.5 to 1 sec and repeat it intermittently. When the solution is sprayed while moving the spray bottle, on the other hand, it is preferred to push the button while moving the spray bottle at a moving speed of from 20 to 100 cm/sec, preferably from 30 to 70 cm/sec, more preferably from 40 to 60 cm/sec.

The aerosol hair cosmetic composition to be used in the present invention is excellent in re-styling performance so that at any time after spraying, a desired style can be completed by holding the hair with fingers and fixing it.

### [Examples]

### Examples 1 to 3

Hair cosmetic compositions (pump sprays) having the compositions as shown in Table 1 were prepared in a conventional manner. The hair cosmetic compositions thus obtained were evaluated for their styling performance, re-styling performance, non-stickiness and non-stiffness. The results are collectively shown in Table 1.

### (Evaluation method)

### (1) Hair styling performance:

A panel of ten experts was asked to use each hair spray for hair styling and organoleptically evaluate its hair styling performance based on the following criteria. The results are indicated by an average of evaluation scores.

### (Evaluation criteria)

5: Excellent.
4: Good.
3: Relatively good.
2: Relatively bad.
1: Bad.

### (2) Re-styling performance:

A panel of ten experts was asked to style their hair with each hair spray and organoleptically evaluate the re-styling performance nine hours after spraying under the environment of 25°C and 65%RH in accordance with the following criteria. The results are indicated by an average of evaluation scores.

### (Evaluation criteria)

5: Excellent.
4: Good.
3: Relatively good.
2: Relatively bad.
1: Bad.

### (3) Non-stickiness and non-stiffness

A panel of ten experts was asked to style their hair with each hair spray and organoleptically evaluate the non-stickiness and non-stiffness in accordance with the following criteria. The results are indicated by an average of evaluation scores.

### (Evaluation criteria)

5: Excellent.
4: Good.
3: Relatively good.
2: Relatively bad.
1: Bad.

**[Table 1]**

| | | Examples | | |
|---|---|---|---|---|
| | | 1 | 2 | 3 |
| A | (Acrylamide/DMAPA acrylate/methoxy PEG methacrylate) copolymer ^{*1} | 4.8 | 3.2 | 3.7 |
| | (Acrylamide/alkyl acrylate/DMAPA acrylate/methoxy PEG methacrylate) copolymer ^{*2} | 1.2 | 0.8 | 0.9 |
| B | Isostearyl glyceryl ether | 4.0 | 6.0 | 5.4 |
| | Lactic acid (90 wt.% aqueous solution) | 0.2 | 0.07 | 0.09 |
| | Ethanol | Balance | Balance | Balance |
| Total | | 100 | 100 | 100 |
| (A)/(B) | | 60/40 | 40/60 | 46/54 |
| Hair styling performance | | 5 | 4 | 4 |
| Re-styling performance | | 4 | 4 | 4 |
| Non-stickiness | | 4 | 3 | 3 |
| Non-stiffness | | 3 | 4 | 5 |

| | | | | |
|---|---|---|---|---|
| *1: Synthesized in accordance with the process described in JP-A-1996-291206. N-tert-butyl acrylamide/dimethyl acrylamide/dimethylaminopropyl acrylamide/methoxy polyethylene glycol (PEG400) methacrylate copolymer <52/25/2/21> (weight ratio) *2: Synthesized in accordance with the process described in JP-A-1990-180911. N-tert-butyl acrylamide/ethyl acrylate/N,N-dimethylaminopropyl acrylamide/polyethylene glycol methacrylate copolymer <55/20/15/10> (weight ratio) | | | | |

### Examples 4 to 12 and Comparative Examples 1 to 5

Stock solutions of a hair spray shown in Table 2 were prepared in a conventional manner. Each of the resulting stock solutions and LPG (0.15 MPa, 20°C) as a propellant were filled in an aerosol container equipped with the below-described valve and button at a (stock solution)/propellant (mass ratio) shown in table 2 to prepare an aerosol hair cosmetic composition. The pressure in the container was 0.23 Mpa at 25°C.
Valve: the stem hole diameter ϕ: 0.41 mm, the bottom hole diameter of a housing ϕ: 0.64 mm × the side hole diameter of a housing ϕ: 0.41 mm.
Button: hole diameter ϕ: 0.46 mm (MB, concave)

### (Precision Valve Japan Co., Ltd.)

The hair cosmetic compositions thus obtained were evaluated in a similar manner to Examples 1 to 3 for hair styling performance, re-styling performance, non-stickiness, and non-stiffness. The results are collectively shown in Table 2.

**[Table 2]**

| Component (wt.%) | | Examples | | | | | | | | | Comparative Examples | | | | |
|---|---|---|---|---|---|---|---|---|---|---|---|---|---|---|---|
| | | 4 | 5 | 6 | 7 | 8 | 9 | 10 | 11 | 12 | 1 | 2 | 3 | 4 | 5 |
| A | (Acrylamide/DMAPA acrylate/methoxy PEG methacrylate) copolymer ^{*1} | 3.7 | 3.7 | 3.7 | 3.7 | 4.6 | | 3.7 | 3.7 | 3.7 | 3.7 | 3.7 | | | 3.7 |
| | (Acrylamide/alkyl aciylate/DMAPA acrylate/methoxy PEG methacrylate) copolymer ^{*2} | 0.9 | 0.9 | 0.9 | 0.9 | | 4.6 | 0.9 | 0.9 | 0.9 | 0.9 | 0.9 | | | 0.9 |
| | Polyvinyl caprolactam ^{*3} | | | | | | | | | | | | | 4.6 | |
| B | Isostearyl glyceryl ether | 5.4 | 5.4 | 5.4 | 0.8 | 0.8 | 0.8 | | | 0.8 | 0.8 | | 5.4 | 5.4 | 0.6 |
| | Oleic acid mono-, di-, and triglycerides ^{*4} | | | | | | | 5.4 | | 1.3 | 1.3 | | | | 0.9 |
| | Oleic acid monoglyceride ^{*5} | | | | 3.3 | 3.3 | 3.3 | | 5.4 | 2.0 | 2.0 | | | | 1.5 |
| | caprylic acid/capric acid triglyceride ^{*6} | | | | 1.3 | 1.3 | 1.3 | | | 1.3 | 1.3 | | | | 0.9 |
| | Dipropylene glycol | | | | | | | | | | 1.5 | | | | 1.5 |
| | Lactic acid (90 wt.% aqueous solution) | 0.09 | 0.09 | 0.09 | 0.09 | 0.06 | 0.47 | 0.09 | 0.09 | 0.09 | 0.09 | 0.09 | | | 0.09 |
| | Ethanol | Balance | Balance | Balance | Balance | Balance | Balance | Balance | Balance | Balance | Balance | Balance | Balance | Balance | Balance |
| Total (stock solution) | | 100 | 100 | 100 | 100 | 100 | 100 | 100 | 100 | 100 | 100 | 100 | 100 | 100 | 100 |
| (A)/(B) (weight ratio) | | 46/54 | 46/54 | 46/54 | 46/54 | 46/54 | 46/54 | 46/54 | 46/54 | 46/54 | 46/54 | 100/0 | 0/100 | 46/54 | 46/54 |
| (C) Propellant | | LPG | LPG | LPG | LPG | LPG | LPG | LPG | LPG | LPG | LPG | LPG | LPG | LPG | LPG |
| (Stock solution)/(C) (weight ratio) | | 47/53 | 33/67 | 40/60 | 40/60 | 40/60 | 40/60 | 40/60 | 40/60 | 40/60 | 40/60 | 40/60 | 40/60 | 40/60 | 54/46 |
| Hair styling performance | | 4 | 3 | 4 | 5 | 5 | 5 | 4 | 4 | 5 | 2 | 5 | 2 | 2 | 2 |
| Re-styling performance | | 4 | 3 | 3 | 5 | 5 | 5 | 4 | 4 | 5 | 1 | 1 | 2 | 2 | 1 |
| Non-stickiness | | 4 | 4 | 5 | 5 | 5 | 4 | 5 | 5 | 5 | 5 | 5 | 3 | 5 | 5 |
| Non-stiffness | | 5 | 5 | 5 | 5 | 4 | 5 | 5 | 5 | 5 | 4 | 1 | 5 | 5 | 4 |

| | | | | | | | | | | | | | | | |
|---|---|---|---|---|---|---|---|---|---|---|---|---|---|---|---|
| *1: Synthesized in accordance with the process described in JP-A-1996-291206. N-tert-butyl acrylamide/dimethyl acrylamide/dimethylaminopropyl acrylamide/methoxyl polyethylene glycol (PEG400) methacrylate copolymer <52/25/2/21> (weight ratio) *2: Synthesized in accordance with the process described in JP-A-1990-180911. N-tert-butyl acrylamide/ethyl acrylate/N,N-dimethylaminopropyl acrylamide/polyethylene glycol methacrylate copolymer <55/20/15/10> (weight ratio) *3: "Luviskol VA64P" (product of BASF) *4: "RHEODOL MO60" (product of Kao) *5: "EXCEL O-95R" (product of Kao) *6**COCONARD MT" (product of Kao) | | | | | | | | | | | | | | | |

In the above Examples, the viscosity of the stock solution was measured using a B type viscometer (rotor with BL adaptor, rotation at 30 rpm for 60 seconds at 30°C) manufactured by Toki Sangyo. The average particle size of liquid droplets thus sprayed is a value corresponding to 50% of the cumulative volume distribution as measured (measurement distance: 15 cm) by spraying the solution directly to laser light at a measuring range R4 (focus distance: 200 mm, particle size measuring range from 0.5/1.8 to 350 µm) by using a laser diffraction particle size distribution analyzer "HELOS SYSTEM" manufactured by Sympatec GmbH, system-Partikel-technik.

### Examples 13 to 17 and Comparative Examples 6 to 9

Stock solutions of a hair spray shown in Table 3 were prepared conventionally. Each of the resulting stock solutions and LPG (0.15 MPa, 20°C) as a propellant were filled in an aerosol container equipped with the below-described valve and button at a (stock solution)/propellant (weight ratio) shown in Table 1. The pressure in the container was 0.23 MPa at 25°C.
Valve: the stem hole diameter ϕ: 0.41 mm, the bottom hole diameter of a housing ϕ: 0.64 mm x the side hole diameter of a housing ϕ: 0.41 mm.
Button: hole diameter ϕ: 0.46 mm (MB, concave) (Precision Valve Japan Co., Ltd.)

The adhesive force of the dry residue was measured in the following manner.

### (Measuring method)

The aerosol hair cosmetic composition is placed in a round-bottom flask. The composition is dried to constant weight at 60°C in a rotary evaporator while reducing pressure to fall within a range of from 15mmHg to 20mmHg, whereby a volatile component is removed and a dry residue is obtained. A 10 wt.% solution of the dry residue in ethanol is prepared. At 25°C and 65%RH, the resulting solution is spread over a PET sheet ("MINERON A-PET Code 506", product of Mineron Kasei Kogyo) and applied uniformly thereto by using a bar coater (No. 120, product of Daiichi Rika), followed by drying at 40°C for 60 minutes. After the thus-obtained sample is maintained at 25°C and 65%RH for 30 minutes, the adhesive force of the dry residue is measured using a tacking tester ("TAC II UC-2006", product of RHESCA) under the following conditions: 8mm diameter probe side base of being made of polypropylene, probe approaching speed of 120 mm/sec, applied pressure of 200 gf, pressure application time of 3 seconds, and pulling-up speed of the probe of 600 mm/sec.
In the invention, four measurement samples are prepared for one dry residue and the adhesive force is measured at five different points. This means that the dry residue is measured at 20 points in total and an average is employed as a measurement value.

Hair styling was performed with the aerosol hair cosmetic compositions in accordance with any one of the following methods (1) to (5):
(1) Dry hair is lifted up with fingertips in such a manner that the width of a hair bundle held with fingertips becomes 10 mm in diameter. Then, the composition is sprayed to a portion of the lifted-up hair which existed on the scalp side and is at least 2 cm distant from the scalp to provide a dry residue after application of 10 mg/cm². The term "dry hair" as used herein means hair having a water content less than a saturated water content of the hair to be sprayed. The saturated water content of the hair is defined by a saturated state of a weight change of hair when the hair is caused to absorb moisture by placing it in an environment having a relative humidity of 98%RH or greater.

(2) Wet hair is lifted up with fingertips in such a manner that the width of a hair bundle held with fingertips becomes 10 mm in diameter. Then, the composition is sprayed to a portion of the lifted-up hair which existed on the scalp side and is at least 2 cm distant from the scalp to provide a dry residue after application of 10 mg/cm². The term "wet hair" as used herein means the hair having a water content equal to or greater than the saturated water content of the hair to be sprayed.

(3) The dry hair is lifted up with fingertips in such a manner that the width of a hair bundle held with fingertips becomes 30 mm in diameter. Then, the composition is sprayed to a portion of the lifted-up hair which existed on the scalp side and is at least 2 cm distant from the scalp to provide a dry residue after application of 10 mg/cm².

(4) The dry hair is lifted up with fingertips in such a manner that the width of a hair bundle held with fingertips becomes 10 mm in diameter. Then, the composition is sprayed to a portion of the lifted-up hair which existed on the scalp side and is at a root position less than 2 cm from the scalp to provide a dry residue after application of 10 mg/cm².

(5) Without lifting up dry hair, the composition is sprayed to the surface of the hair so that the dry residue after application to the hair becomes 10 mg/cm².

With regard to the thus-styled hair, each composition was evaluated for its styling performance, re-styling performance, non-stickiness, and non-stiffness in a similar manner to that employed in Examples 1 to 3. Further, the composition was evaluated for a fluffy feel provided to the hair. The results are collectively shown in Table 3.

### (Evaluation method)

### Fluffy feel:

A panel of ten experts was asked to style their hair with each hair spray and organoleptically evaluate its ability to keep the hair greatly fluffed as compared with the original hair style in accordance with the following criteria. The results are indicated by an average of evaluation scores.

### (Evaluation criteria)

5: Excellent.
4: Good.
3: Relatively good.
2: Relatively bad.
1: Bad.

**[Table 3]**

| Component (wt.%) | | Examples | | | | | Comparative Examples | | | |
|---|---|---|---|---|---|---|---|---|---|---|
| | | 13 | 14 | 15 | 16 | 17 | 6 | 7 | 8 ^{*16} | 9 |
| A | Hair styling polymer 1 ^{*11} | 3.8 | | | | | 3.8 | 10 | | |
| | Hair styling polymer 2 ^{*12} | | 3.8 | 3.8 | 3.8 | 3.8 | | | | 3.8 |
| | Hair styling polymer 3 ^{*13} | | 0.95 | 0.95 | 0.95 | 0.95 | | | | 0.95 |
| | Hair styling polymer 4 ^{*14} | | | | | | | | 1.0 | |
| | Lactic acid (90 wt.%) | | 0.135 | 0.135 | 0.135 | 0.135 | | 0.13 | | 0.135 |
| | AMP-100 ^{*15} | | | | | | | | | |
| | Acetyl monoethanolamine | | | | | | | | 2.0 | |
| B | Isostearyl glyceryl ether | 5.7 | 0.79 | 0.79 | 0.79 | 0.79 | 0.95 | | 1.0 | 0.79 |
| | Oleic acid monoglyceride | | 2.85 | 2.85 | 2.85 | 2.85 | | | | 2.85 |
| | Capric acid triglyceride | | 1.11 | 1.11 | 1.11 | 1.11 | | | | 1.11 |
| | Dipropylene glycol | | | | | | 4.8 | | | |
| | Ethanol | Balance | Balance | Balance | Balance | Balance | Balance | Balance | Balance | Balance |
| Total (stock solution) | | 100 | 100 | 100 | 100 | 100 | 100 | 100 | 100 | 100 |
| Adhesive force of dry residue (gf/cm²) | | 25.88 | 113.4 | 113.4 | 113.4 | 113.4 | 5.3 | 1.4 | 18.5 | 113.4 |
| Stock solution/propellant (weight ratio) | | 60/40 | 60/40 | 60/40 | 60/40 | 60/40 | 60/40 | 60/40 | 60/40 | 60/40 |
| Styling method | | (1) | (1) | (2) | (3) | (4) | (1) | (1) | (1) | (5) |
| Viscosity of stock solution (mPa·s) | | 7.5 | 9.6 | 9.6 | 9.6 | 9.6 | 6.4 | 7.2 | 6.1 | 9.6 |
| spraying particle size (µm) | | 24 | 26 | 26 | 26 | 26 | 29 | 28 | 25 | 26 |
| Weight loss by spraying | | 4.2 | 3.7 | 3.7 | 3.7 | 3.7 | 3.6 | 3.8 | 3.7 | 3.7 |
| Hair styling performance | | 3 | 5 | 4 | 3 | 3 | 2 | 5 | 2 | 2 |
| Re-styling performance | | 3 | 5 | 4 | 3 | 3 | 2 | 1 | 2 | 2 |
| Non-stickiness | | 4 | 4 | 3 | 2 | 4 | 5 | 4 | 4 | 1 |
| Non-stiffness | | 4 | 4 | 4 | 4 | 4 | 5 | 1 | 4 | 4 |
| Fluffy feel | | 4 | 5 | 3 | 2 | 2 | 2 | 2 | 1 | 1 |

**[Table 4]**

| |
|---|
| *11: (vinyl pyrrolidone/vinyl acetate) copolymer ("Luviskol VA64P", product of BASF) |
| *12*: Synthesized in accordance with the process described in JP-A-1996-291206. t-Butyl acrylamide/dimethyl acrylamide/dimethylaminopropyl acrylamide/methoxy polyethylene glycol (PEG400) methacrylate copolymer <52/25/2/21> (weight ratio) |
| *13: Synthesized in accordance with the process described in JP-A-1990-180911. Alkyl acrylamide/acrylate/alkylaminoalkyl acrylamide/polyethylene glycol methacrylate copolymer <55/20/15/10> (weight ratio) |
| *14: Acrylic acid/ethyl acrylate/N-t-butyl acrylamide ("Ultrahold 8", product of BASF) |
| *15: Aminomethyl propanol (product of Dow Chemical) |
| *16: The formulation of Example 2 described in JP-A-1999-116443 was, together with a propellant, filled in an aerosol container. |

### Examples 18 to 42

The stock solutions of a hair spray shown in Tables 5 to 7 are prepared in a conventional manner. Each of the resulting stock solutions is, together with LPG (0.15 MPa, 20°C) as a propellant, filled in an aerosol container equipped with the below-described valve and button at a (stock solution)/propellant (mass ratio) of 60/40. The pressure in the container is 0.23 Mpa at 25°C.
Valve: the stem hole diameter ϕ: 0.41 mm, the bottom hole diameter of a housing ϕ: 0.64 mm x the side hole diameter of a housing ϕ: 0.41 mm.
Button: hole diameter ϕ: 0.46 mm (MB, concave)

### (Precision Valve Japan Co., Ltd.)

Hair styling with the resulting aerosol hair cosmetic compositions is performed in accordance with the method (1) shown in Examples 13 to 17.
Any of the aerosol hair cosmetic compositions has an adhesive force of 20 gf/cm² or greater when measured using the method defined by the present invention. Hair styling with the composition can achieve a fluffy and light hold and at the same time, the finished hair style can be retained for long hours. Even if the hair style collapses, it can be styled again.

**[Table 5]**

| Component (wt.%) | | Examples | | | | | | | | | | | | |
|---|---|---|---|---|---|---|---|---|---|---|---|---|---|---|
| | | 18 | 19 | 20 | 21 | 22 | 23 | 24 | 25 | 26 | 27 | 28 | 29 | 30 |
| A | Hair styling polymer 1 ^{*11} | | | | 3.8 | 3.8 | | | | | | | | |
| | Hair styling polymer 2 ^{*12} | | | | | | | 4.75 | 4.75 | 4.75 | 4.75 | 4.75 | 4.75 | |
| | Hair styling polymer 3 ^{*13} | | | | | | | | | | | | | 4.25 |
| | Hair styling polymer 5 ^{*17} | | | | | | 3.8 | | | | | | | |
| | Hair styling polymer 6 ^{*18} | 3.8 | | | | | | | | | | | | |
| | Hair styling polymer 7 ^{*19} | | 3.8 | | | | | | | | | | | |
| | Hair styling polymer 8 ^{*20} | | | 2.85 | | | | | | | | | | |
| | Lactic acid (90 wt.%) | | | | | | | 0.02 | 0.02 | 0.02 | 0.02 | 0.02 | 0.02 | 0.17 |
| B | Isostearyl glyceryl ether ^{*21} | 5.7 | 5.7 | 6.65 | | | | 4.75 | | | | | | 4.25 |
| | Polyoxyethylene tridecyl ether ^{*22} | | | | | | | | 4.75 | | | | | |
| | Oleic acid monoglyceride/diglyceride/triglyceride ^{*23} | | | | 5.7 | | | | | 4.75 | | | | |
| | Polyoxyethylene (EO=4) lauryl ether ^{*24} | | | | | | | | | | 4.75 | | | |
| | Polyoxyethylene (EO=9) oleyl ether ^{*25} | | | | | | | | | | | 4.75 | | |
| | Sorbitan fatty acid ester ^{*26} | | | | | 5.7 | | | | | | | | |
| | EO/PO adduct of sorbitan fatty acid easter ^{*27} | | | | | | | | | | | | | |
| | Polyoxyethylene (EO=20) sorbitan monostearate ^{*28} | | | | | | | | | | | | | |
| | Polyethylene glycol (Mw=400) ^{*29} | | | | | | 5.7 | | | | | | 4.75 | |
| | Ethanol | Balance | Balance | Balance | Balance | Balance | Balance | Balance | Balance | Balance | Balance | Balance | Balance | Balance |
| Total (stock solution) | | 100 | 100 | 100 | 100 | 100 | 100 | 100 | 100 | 100 | 100 | 100 | 100 | 100 |

**[Table 6]**

| |
|---|
| *11: (vinyl pyrrolidone/vinyl acetate) copolymer ("Luviskol VA64P", product of BASF) |
| *12*: Synthesized in accordance with the process described in JP-A-1996-291206. t-Butyl acrylamide/dimethyl acrylamide/dimethylaminopropyl acrylamide/methoxy polyethylene glycol (PEG400) methacrylate copolymer <52/25/2/21> (mass ratio) |
| *13: Synthesized in accordance with the process described in JP-A-1990-180911. Alkyl acrylamide/acrylate/alkylaminoalkyl acrylamide/polyethylene glycol methacrylate copolymer <55/20/15/10> (mass ratio) |
| *17: (Octyl acrylamide/hydroxypropyl acrylate/butylaminoethyl methacrylate) copolymer ("AMPHOMER 28-4910", product of National Starch & Chemical) |
| *18: Acrylic acid/acrylate copolymer, 40% ethanol solution ('PLAS CIZE L9540B", product of Goo Chemical). Indicatedas a solid base. |
| *19: Acrylic acid/acrylate copolymer, 40% ethanol solution ("PLAS CIZE L9909B", product of Goo Chemical). Indicatedas a solid base. |
| *20: (Methacryloyloxyethylcarboxybetaine/alkyl methacrylate) copolymer, 30% ethanol solution ("Yukaformer M-75", product of Mitsubishi Chemical). Indicatedas a solid base. |
| *21: "PENETOL GE-IS" (product of Kao) |
| *22: "SOFTANOL 90" (product of Sanyo Chemical) |
| *23: "RHEODOL MO-60" (product of Kao) |
| *24: "EMULGEN 104P" (product of Kao) |
| *25: "EMULGEN 409P" (product of Kao) |
| *26: "RHEODOL SUPER SP-L10" (product of Kao) |
| *27: "RHEODOL SUPER TW-L120" (product of Kao) |
| *28: "RHEODOL TW-S120V" (product of Kao) |
| *29: "PEG-400" (product of Nippon Shokubai) |

**[Table 7]**

| Component (wt.%) | | Examples | | | | | | | | | | | |
|---|---|---|---|---|---|---|---|---|---|---|---|---|---|
| | | 31 | 32 | 33 | 34 | 35 | 36 | 37 | 38 | 39 | 40 | 41 | 42 |
| A | Hair styling polymer 1 ^{*11} | | | | | | | | | | | | |
| | Hair styling polymer 2 ^{*12} | | | | | | | | | | | | |
| | Hair styling polymer 3 ^{*13} | | | | | | | | | | | | |
| | Hair styling polymer 5 ^{*17} | | | | | | | | | | | | |
| | Hair styling polymer 6 ^{*18} | 3.8 | 3.8 | 3.8 | 3.8 | 3.8 | 3.8 | 3.8 | 3.8 | | | | |
| | Hair styling polymer 7 ^{*19} | | | | | | | | | 3.8 | 3.8 | | |
| | Hair styling polymer 8 ^{*20} | | | | | | | | | | | 2.85 | 2.85 |
| | Lactic acid (90 wt.%) | | | | | | | | | | | | |
| B | Isostearyl glyceryl ether ^{*21} | | | | | | | | | | | | |
| | EO/PO alkyl ether ^{*22} | 5.7 | | | | | | | | | | | |
| | Oleic acid monoglyceride/diglyceride/triglyceride ^{*23} | | 5.7 | | | | | | | 5.7 | | 6.65 | |
| | Polyoxyethylene (EO=4) lauryl ether ^{*24} | | | 5.7 | | | | | | | | | |
| | Polyoxyethylene (EO=9) oleyl ether ^{*25} | | | | 5.7 | | | | | | | | |
| | Sorbitan fatty acid ester ^{*26} | | | | | 5.7 | | | | | | | 6.65 |
| | EO/PO adduct of sorbitan fatty acid ester ^{*27} | | | | | | 5.7 | | | | | | |
| | Polyoxyethylene (EO=20) sorbitan monostearate ^{*28} | | | | | | | 5.7 | | | | | |
| | Polyethylene glycol (Mw=400) ^{*29} | | | | | | | | 5.7 | | 5.7 | | |
| | Ethanol | Balance | Balance | Balance | Balance | Balance | Balance | Balance | Balance | Balance | Balance | Balance | Balance |
| Total (stock solution) | | 100 | 100 | 100 | 100 | 100 | 100 | 100 | 100 | 100 | 100 | 100 | 100 |

## Claims

1. A hair cosmetic composition comprising the following components (A) and (B):
(A) is/are one or more copolymers selected from the group consisting of the following components (A1) and (A2),
wherein
(A1) is a copolymer of:
(a) from 30 to 80 wt.% of a (meth)acrylamide monomer represented by the following formula (1): 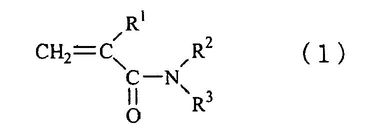 wherein, R¹ represents a hydrogen atom or a methyl group and R² and R³ may be the same or different and each represents a hydrogen atom or an alkyl group having from 4 to 12 carbon atoms, with the proviso that R² and R³ do not represent a hydrogen atom simultaneously;
(b) from 2 to 50 wt.% of a (meth)acrylamide monomer represented by the following formula (2): 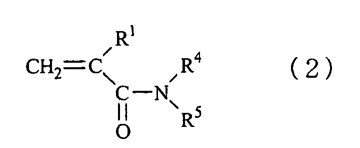 wherein, R¹ has the same meaning as defined above, R⁴ and R⁵ may be the same or different and each represents a hydrogen atom or an alkyl group having from 1 to 3 carbon atoms;
(c) from 0 to 30 wt.% of a (meth)acrylate monomer or (meth)acrylamide monomer represented by the following formula (3): 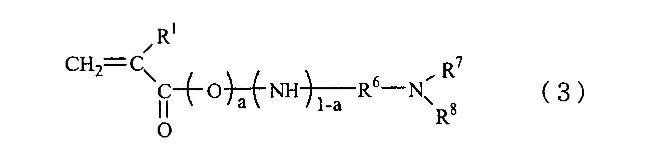 wherein R¹ has the same meaning as defined above, R⁶ represents an alkylene group having 2 or 3 carbon atoms, R⁷ and R⁸ may be the same or different and each represents a methyl or ethyl group, and "a" denotes a number of 0 or 1;
and
(d) from 0 to 40 wt.% of a (meth)acrylate monomer represented by the following formula (4): 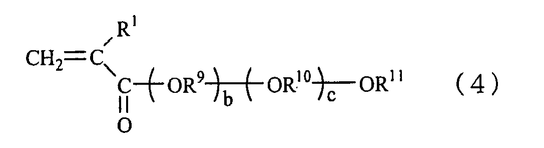
wherein R¹ has the same meaning as defined above, R⁹ and R¹⁰ may be the same or different and each represents an alkylene group having from 2 to 4 carbon atoms, R¹¹ represents a hydrogen atom, an alkyl group having from 1 to 10 carbon atoms, or a phenyl group, and b and c each denotes a number of from 0 to 50, with the proviso that b and c do not stand for 0 simultaneously; and
(A2) is a copolymer of:
(a) from 30 to 80 wt.% of a (meth)acrylamide monomer represented by the following formula (5): 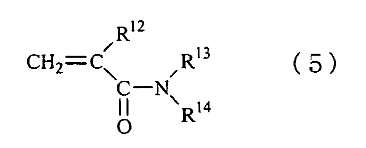 wherein, R¹² represents a hydrogen atom or a methyl group,
and R¹³ and R¹⁴ may be the same or different and each represents a hydrogen atom or an alkyl group having from 4 to 12 carbon atoms or R¹³ and R¹⁴ are coupled together to form a ring with a nitrogen atom adjacent thereto;
(b) from 5 to 45 wt.% of a (meth)acrylate monomer represented by the following formula (6): 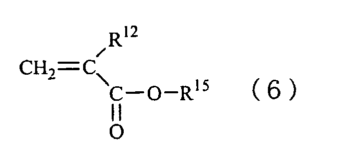 wherein, R¹² has the same meaning as defined above and R¹⁵ represents an alkyl group having from 1 to 4 carbon atoms;
(c) from 2 to 30 wt.% of a (meth)acrylate monomer or (meth)acrylamide monomer represented by the following formula (7): 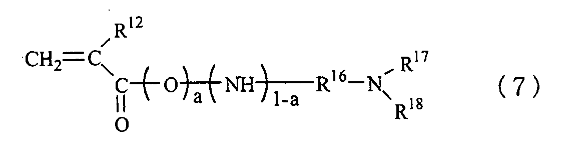 wherein, R¹² has the same meaning as defined above, R¹⁶ represents an alkylene group having 2 or 3 carbon atoms, R¹⁷ and R¹⁸ may be the same or different and each represents a methyl or ethyl group, and "a" denotes a number of 0 or 1; and
(d) from 0 to 30 wt.% of a (meth)acrylate monomer represented by the following formula (8): 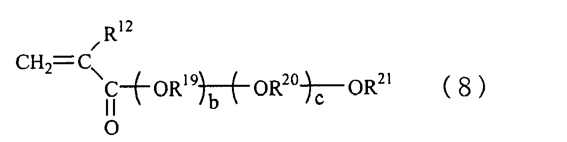
wherein, R¹² has the same meaning as defined above, R¹⁹ and R²⁰ may be the same or different and each represents an alkylene group having from 2 to 4 carbon atoms, R²¹ represents a hydrogen atom or a methyl group, and b and c each denotes a number of from 0 to 50 with the proviso that b and c do not stand for 0 simultaneously; and
(B) is at least one or more selected from the group consisting of long-chain alkyl glyceryl ethers, fatty acid monoglycerides, fatty acid diglycerides, and fatty acid triglycerides;
and wherein the cosmetic composition contains a compound having two or more hydroxyl groups in the molecule, the compound having a molecular weight of from 62 to 1000, and being in liquid form at 30°C, in an amount less than 1 wt.%.

2. The hair cosmetic composition according to Claim 1, wherein a weight ratio of Component (A) to Component (B), that is, a weight ratio of (A)/(B) is from 65/35 to 35/65.

3. The hair cosmetic composition according to Claim 1, wherein the hair cosmetic composition is an aerosol hair cosmetic composition further comprising (C) a propellant.

4. The hair cosmetic composition according to Claim 3, wherein a weight ratio of the stock solution to Component (C), that is, a weight ratio of a (stock solution)/(C) is from 50/50 to 30/70.

5. A hair styling method, the method comprising the steps of lifting up hair and spraying, to the inside thereof, an aerosol hair cosmetic composition, the composition containing (A') a hair styling polymer and (B') a plasticizer, wherein the composition has an adhesive force of 20 gf/cm² or greater as measured by the following method.
(Measuring method)
The aerosol hair cosmetic composition is placed in a round-bottom flask. The composition is dried to constant weight at 60°C in a rotary evaporator while reducing the pressure to fall within a range of from 15 mmHg to 20 mmHg, whereby a volatile component is removed and a dry residue is obtained. A 10 wt.% solution of the dry residue in ethanol is prepared. At 25°C and 65%RH, the resulting solution is spread over a PET sheet and applied uniformly thereto by using a bar coater, followed by drying at 40°C for 60 minutes to obtain a measurement sample. After the sample is maintained at 25°C and 65%RH for 30 minutes, the adhesive force of the dry residue is measured using a tacking tester under the following conditions: 8mm diameter probe side base of being made of polypropylene, probe approaching speed of 120 mm/sec, applied pressure of 200 gf, pressure application time of 3 seconds, and pulling-up speed of the probe of 600 mm/sec.

6. The hair styling method according to Claim 5, wherein the aerosol hair cosmetic composition is sprayed to dry hair.

7. The hair styling method according to Claim 5 or 6, wherein the hair is lifted up with fingertips.

8. The hair styling method according to any one of Claims 5 to 7, wherein the composition is sprayed to a portion of the hair at least 2 cm distant from the scalp.
